# FASCICULE DE BREVET EUROPEEN

(11) **EP 2 475 463 B1**
(45) Date de publication et mention de la délivrance du brevet: **14.08.2013**
(21) Numéro de dépôt: 10768999.4
(22) Date de dépôt: 09.09.2010
(51) Int. Cl.: B05B 1/30, B05B 11/04, B05B 11/00, A61F 9/00, B65D 47/18, B65D 47/20

(54) **DISPOSITIF DE DISTRIBUTION DE LIQUIDE**
VORRICHTUNG ZUR AUSGABE EINER FLÜSSIGKEIT
DEVICE FOR DISPENSING LIQUID

(30) Priorité: 11.09.2009 FR 0956279
(43) Date de publication de la demande: 18.07.2012
(73) Titulaire: Rexam Healthcare La Verpillière, 38290 La Verpillière (FR)
(72) Inventeur: PAINCHAUD, Gaetan, F-69340 Francheville (FR); GREVIN, Guillaume, F-38080 L'isle D'abeau (FR); DECOCK, Thierry, F-75013 Paris (FR); JULIA, Xavier, F-38090 Villefontaine (FR)
(74) Mandataire: Thiollier, Clémence-Olivia Laure Marie
(86) Numéro de dépôt international: PCT/FR2010/051878
(87) Numéro de publication internationale: WO 2011/030063

(56) Documents cités:
- EP-A1- 1 520 797
- WO-A1-2004/069679
- US-A1- 2007 113 841

## Description

L'invention concerne le domaine de la distribution de liquide, notamment sous forme de gouttes dans le domaine pharmaceutique, par exemple du liquide ophtalmique ou auriculaire.

On connaît déjà des dispositifs de distribution de liquide munis d'un réservoir et d'un embout de distribution monté sur le réservoir, cet embout étant muni d'un support et d'un élément de fermeture. Par exemple, dans le document US 2007/0210115, l'embout de distribution comprend un support à l'intérieur duquel un élément de fermeture est déplaçable entre une configuration de blocage et une configuration de passage du liquide. Cet élément de fermeture comporte par ailleurs à son extrémité distale, au voisinage immédiat de l'orifice de libération de liquide, une forme de réduction du débit de liquide. Cette forme de réduction comprend un canal de forme hélicoïdale, de faible section et permettant de dévier le liquide pour générer une perte de charges lorsqu'un utilisateur appuie sur le réservoir. Grâce à cette forme de réduction de débit, on évite de distribuer le liquide sous forme de jets.

Les inventeurs à l'origine de la présente invention ont constaté qu'une difficulté réside dans le fait que la forme de réduction du débit de liquide est réalisée dans la même partie que la forme assurant le blocage ou le passage du liquide. En effet, une telle configuration requiert que la valve et le support aient des dimensions très précises, car il faut autoriser à la fois un déplacement de la valve par rapport au support, pour assurer le passage de liquide, et une coopération entre la forme de déviation et le support suffisamment étroite pour obtenir un canal de déviation ne présentant pas de fuites, auquel cas l'effet de réduction du débit n'aurait pas lieu. Or, il est délicat de réaliser des pièces requérant une telle précision de dimensions.

La présente invention a notamment pour but de proposer un dispositif de distribution assurant une réduction de débit de liquide tout en garantissant un fonctionnement satisfaisant de distribution de liquide.

A cet effet, l'invention a notamment pour objet un dispositif de distribution de liquide selon la revendication 1.

On propose de réaliser la fonction de réduction de débit par l'intermédiaire d'une troisième pièce, distincte de la valve et du support, c'est-à-dire séparable de la valve et du support, ou du moins séparée des deux autres avant l'assemblage du dispositif. En faisant intervenir une pièce à part pour assurer la limitation de débit de liquide, on dissocie les parties de l'embout qui assurent l'étanchéité du dispositif, par blocage ou passage de liquide, et celles qui assurent la réduction de débit. En effet, la réduction étant assurée par une troisième pièce, ne se situant pas au voisinage direct de la partie d'étanchéité réalisée par coopération de la valve et du support, la fonction d'étanchéité peut être assurée indépendamment de la fonction de réduction du débit. Aussi, chaque fonction est assurée de façon optimale et fiable. Alors que l'on pourrait s'attendre à ce que l'assemblage du dispositif soit plus compliqué, du fait que l'on ajoute une nouvelle pièce rapportée, l'assemblage se trouve simplifié, du fait que la zone de réduction de débit n'est plus dépendante de la forme de la valve et du support formant l'étanchéité du dispositif. En conséquence il est plus aisé de modifier la longueur ou le diamètre de cette forme de réduction de débit. En outre, du fait que la réduction de débit n'est pas assurée dans la zone d'étanchéité de l'embout, cette dernière peut présenter une longueur moins importante, et permettre ainsi de réaliser des embouts plus compacts.

Comme les fonctions sont dissociées, la fabrication est facilitée, puisque des variations de dimension des pièces ont moins d'effet sur le fonctionnement du dispositif. Ceci est avantageux car, pour des niveaux de précision trop élevés, des difficultés industrielles se présentent, telles que la fragilité des outils ou la casse du moule.

Par ailleurs, un avantage particulier réside dans le fait qu'il est facilement possible d'adapter le dispositif en fonction du type de liquide qui est distribué. En effet, selon la viscosité du liquide, le canal de réduction du débit doit présenter une configuration particulière. Par exemple, on prévoira un canal de réduction de liquide ayant un diamètre plus grand pour les liquides très visqueux. Or, comme l'organe de réduction de débit est une pièce à part, pour adapter l'embout de distribution en fonction du type de liquide à distribuer, il suffit de modifier uniquement la forme de l'organe de réduction de débit, sans avoir à modifier pour autant la forme de la valve ou du support, qui peuvent donc avoir une forme standard. En outre, comme la diminution de débit est assurée par une pièce à part, on peut fournir à la fois des dispositifs présentant la fonction de diminution de débit et des dispositifs ne la présentant pas, la seule différence entre ces dispositifs étant la présence ou non de l'organe de réduction de débit.

On notera que l'adaptation du dispositif en fonction de la viscosité du produit est particulièrement intéressante, du fait que les produits tels que des produits ophtalmiques ont tendance à devenir de plus en plus visqueux, notamment pour les produits ne comprenant pas d'agent conservateur.

On entend par "réduction de débit" le fait de diminuer la vitesse d'écoulement du liquide pour une même pression appliquée par l'utilisateur sur le réservoir. Cette réduction de débit est générée de préférence par un canal ayant une section plus faible que d'autres canaux d'écoulement prévus sur le dispositif et/ou s'étendant sur une grande longueur et/ou ayant un axe transversal.

Avantageusement, on pourrait profiter de l'organe de réduction de débit pour combler un espace du dispositif, et ainsi limiter un volume mort se situant dans l'embout de distribution, le volume mort étant source de développement de bactéries.

Le dispositif de distribution peut en outre comporter l'une ou plusieurs des caractéristiques suivantes.
- Le support comporte un logement de réception de l'organe de réduction de débit délimitant, avec ce dernier, le canal de réduction de débit. Ce logement peut par exemple être disposé à l'opposé de la partie du support qui assure l'étanchéité du dispositif, de façon à limiter encore plus les interactions entre la limitation de débit et l'étanchéité. De préférence, ce logement de réception a une forme sensiblement cylindrique, délimitant une cavité de volume sensiblement complémentaire au volume de l'organe de réduction de débit.
- L'organe de réduction de débit a une forme sensiblement cylindrique, la forme de déviation étant réalisée sur la périphérie extérieure de l'organe de réduction de débit et comprenant une gorge hélicoïdale. Cette forme hélicoïdale du canal fournit une direction d'écoulement transverse, non parallèle à la direction d'éjection du liquide, tout en fournissant une longueur de canal relativement grande, ce qui permet d'obtenir une perte de charges tout en prenant une place minimale dans le dispositif.
- L'organe de réduction de débit a une forme sensiblement cylindrique, la forme de déviation étant réalisée sur la périphérie extérieure de l'organe de réduction de débit et comprenant une gorge définissant des portions anguleuses. Ces portions anguleuses permettent de définir une trajectoire avec des changements de direction nets, ce qui perturbe l'écoulement de fluide et entraîne une plus grande perte de charges.
- L'organe de réduction de débit a une forme sensiblement cylindrique, la forme de déviation étant réalisée sur la périphérie extérieure de l'organe de réduction de débit et comprenant une gorge rectiligne, de préférence sensiblement parallèle à l'axe du cylindre de l'organe de réduction de débit. Du fait des caractéristiques géométriques de la gorge, notamment des dimensions de sa section, l'écoulement de fluide est perturbé et entraine une perte de charges qui peut s'avérer suffisante pour réduire le débit.
- L'organe de réduction de débit a une forme sensiblement cylindrique, la forme de déviation étant réalisée sur la périphérie extérieure de l'organe de réduction de débit et comprenant un réseau de plusieurs gorges comprenant au moins un noeud de confluence des gorges. Les écoulements de fluide des différentes gorges se rencontrent au niveau du noeud de confluence. Cela crée une zone de turbulence qui entraine une perte de charge élevée.
- La valve comprend une partie de fixation permanente de la valve sur le support, et une partie ayant sensiblement la forme d'un disque, à partir de laquelle fait saillie la partie cylindrique.
- La valve comprend un matériau élastomère, ainsi qu'éventuellement une partie rigide, par exemple servant d'appui à un ressort rappelant la valve en position de blocage de liquide.
- Le support comprend en outre un canal de passage d'air dans le réservoir, muni d'un filtre hydrophobe.

Comme on peut le constater, le support peut participer à un certain nombre de fonctions dans l'embout, à savoir la fonction d'étanchéité, la fonction de réduction de liquide, la fonction de fixation de la valve et/ou la fonction de passage d'air pour aérer le réservoir.

L'invention a par ailleurs pour objet un lot de deux dispositifs tels que présentés ci-dessus, chacun des deux dispositifs présentant une valve et un support de même forme et un organe de réduction de débit de forme différente. Par exemple, le canal de réduction de débit peut avoir une section différente, une longueur différente ou encore une forme différente.

L'invention sera mieux comprise à la lecture de la description qui va suivre, donnée uniquement à titre d'exemple, et faite en se référant aux dessins annexés dans lesquels :
- la figure 1 est une vue en coupe longitudinale d'un dispositif de distribution selon un mode de réalisation ;
- la figure 2 est une vue en coupe longitudinale partielle d'un dispositif de distribution selon un deuxième mode de réalisation ; et
- les figures 3a à 3f sont des vues de côté d'un organe de réduction de débit selon différentes variantes.

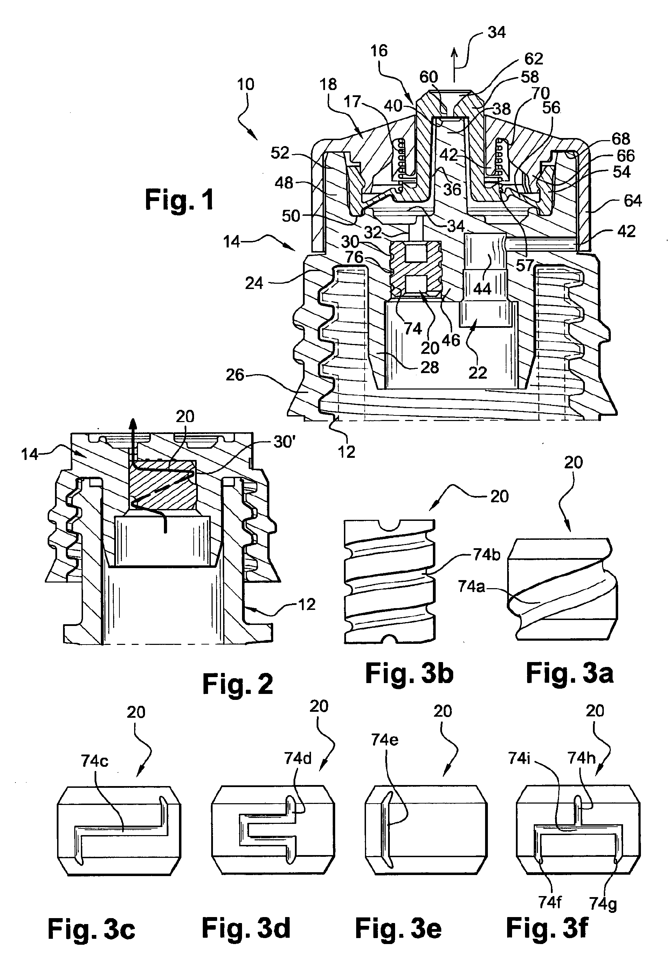

On a représenté sur la figure 1 un embout 10 de distribution de liquide sous forme de gouttes, destiné à être monté par vissage sur le col d'un réservoir 12 représenté en pointillés. Ce réservoir 12 est un réservoir de stockage de liquide pharmaceutique, tel que du liquide ophtalmique ou auriculaire. La distribution de liquide se fait par pression, de la part d'un utilisateur, sur le corps du réservoir 12, ce dernier pouvant présenter une certaine élasticité pour reprendre sa forme initiale après la pression exercée par l'utilisateur.

L'embout de distribution 10 comprend, dans cet exemple, un support 14, une valve de distribution 16, un ressort 17, un capot 18, un organe 20 de réduction de débit et un filtre hydrophobe 22.

La valve 16, le support 14 et l'organe de réduction de débit 20 constituent des pièces distinctes, c'est-à-dire rapportées les unes par rapport aux autres, c'est-à-dire séparées avant montage.

Le support 14 comprend, dans cet exemple, une partie 24 de fixation sur le réservoir, disposée à l'extrémité proximale du support 14. Cette partie 24 comprend une jupe externe 26, taraudée de façon à être vissée sur le col du réservoir 12. La partie de fixation 24 comporte en outre une jupe interne 28 de forme tubulaire, permettant d'assurer l'étanchéité entre le réservoir 12 et l'embout de distribution 10.

Le support 14 comprend également un logement 30 de réception de l'organe de réduction de débit 20, délimitant une cavité sensiblement cylindrique, cette cavité débouchant à son extrémité proximale sur le réservoir 112 et à son extrémité distale sur un premier canal 32 de passage de liquide, ménagé dans le support 14 et s'étendant dans la direction longitudinale du dispositif, correspondant ici à la direction d'éjection du liquide illustrée par la flèche 34. Ce premier canal 32 débouche sur une cavité intermédiaire 34, débouchant elle-même sur un deuxième canal 36 de passage de liquide.

Le support 14 comporte par ailleurs une partie centrale d'étanchéité 38, de forme sensiblement cylindrique et s'étendant dans la direction distale, opposée à la jupe interne 28. La partie 38 porte, sur son extrémité distale, une surface 40 d'appui de la valve 16 pour bloquer le passage de liquide en configuration de blocage. Dans cet exemple, la surface d'appui 40 prend la forme d'un bourrelet annulaire.

Le support 14 comporte également, dans cet exemple, un canal 42 de passage d'air dans le réservoir 12, débouchant sur un logement 44 dans lequel est reçu le filtre hydrophobe 22. Le logement 44 est juxtaposé au logement 30 de réception de l'organe 20 de réduction de débit, en étant séparé par une paroi centrale 46, s'étendant dans une direction opposée à la partie d'étanchéité 38.

Enfin, le support 14 comprend une partie 48 de fixation de la valve 16 sur le support 14. Cette partie 48 fait également office de partie de fixation du capot 18 sur le support 14. Elle comprend une gorge annulaire 50 délimitée en périphérie par une paroi annulaire 52. La gorge annulaire 50 est par ailleurs délimitée, sur sa périphérie intérieure, par une nervure annulaire réalisée sur une paroi formant sensiblement un disque, traversée par le canal 32 et délimitant la cavité 34.

La valve 16 peut prendre une configuration de blocage et une configuration de passage du liquide, par coopération avec le support 14. Elle est réalisée, dans cet exemple, dans un matériau élastomère. Selon un autre exemple, seule une partie de la valve 16 est réalisée dans un matériau élastomère, l'autre partie étant réalisée dans un matériau plus rigide, pouvant servir d'appui au ressort 17. La valve 16 comprend une partie 54 de fixation au support 14, formant une jupe de forme sensiblement tubulaire. Cette partie de fixation 54 est reliée à un voile 56 ayant sensiblement la forme d'un disque et à partir duquel une partie centrale 58 sensiblement cylindrique fait saillie. Le voile 56 comporte également un siège 57 d'appui du ressort 17. La partie 58 définit une cavité intérieure de forme sensiblement cylindrique, complémentaire du pion 38. Le pion 38 et la partie cylindrique 58 sont coaxiaux et délimitent ensemble le canal de passage 36. Ce canal de passage 36 débouche sur un canal de sortie 60 réalisé dans l'extrémité distale de la valve 16, débouchant lui-même sur une forme 62 de formation de gouttes.

Le capot 18 comprend une partie 64 de fixation sur le support 14, annulaire, ainsi qu'une autre partie annulaire 66, coaxiale avec la partie 64, de façon à définir une gorge 68 dans laquelle la paroi annulaire 52 est encastrée. Le capot 18 comprend par ailleurs un siège 70 d'appui du ressort 17, prolongé sur sa périphérie intérieure par une paroi annulaire 72, traversée par la partie 58 et ayant une fonction de centrage de la partie 58 de la valve 16.

L'organe 20 de réduction de débit comprend une forme 74 de déviation de liquide, délimitant, avec le logement 30, un canal 76 de réduction de débit.

L'organe de réduction de débit 20 peut prendre par exemple l'une des formes illustrées sur les figures 3a à 3f. Il a une forme sensiblement cylindrique, la forme de déviation 74 étant réalisée sur sa périphérie extérieure, formant un évidement de la périphérie.

Sur les exemples des figures 3a, 3b, la forme 74 est définie par une gorge hélicoïdale 74a, 74b qui fait le tour du cylindre 20, de préférence plusieurs tours si le liquide est peu visqueux. La gorge 74a a une section plus grande que la gorge 74b, et est adaptée à des liquides de viscosité plus importante que la gorge 74b.

Les organes 20 des figures 3c et 3d illustrent d'autres types de gorges 74c, 74d, qui ne font pas tout le tour de la partie cylindrique de l'organe 20 et qui définissent des portions anguleuses, ici des angles droits, ayant pour effet de diminuer la pression du liquide qui s'écoule. Éventuellement, l'organe 20 peut comprendre, sur d'autres parties de sa paroi périphérique, des gorges similaires aux gorges 74c, 74d, ce qui a pour effet d'augmenter le débit du liquide.

L'organe 20 de la figue 3e illustre une gorge 74e rectiligne sensiblement parallèle à l'axe de la partie cylindrique. En variante, l'organe 20 comprend plusieurs gorges 74e réparties sur l'ensemble de la paroi périphérique de l'organe 20. Dans une autre variante, le ou les gorges 74e sont inclinées par rapport à l'axe du cylindre.

L'organe 20 de la figure 3f illustre un réseau de gorges 74f-h comprenant un noeud 74i de confluence des gorges 74f-h. Le réseau comprend des gorges 74f-g, dites amont, situées en amont du noeud 74i et au moins un gorge 74h, dite aval, située en aval du noeud 74i. En variante, le réseau comprendra plus de deux gorges amont 74f-g, plus d'une gorge aval 74h et plus d'un noeud 74i.

Le fonctionnement du dispositif de la figure 1 va à présent être décrit.

Au repos, c'est-à-dire lorsque aucun utilisateur n'appuie sur le réservoir 12, la valve 16 est en configuration de blocage du liquide, c'est-à-dire qu'elle est en appui sur la surface 40, du fait de sa fixation permanente sur le support 14, exerçant une contrainte élastique sur la valve, et sous l'effet de la pression effectuée par le ressort 17.

Lorsqu'un utilisateur appuie sur le réservoir 12, il exerce une pression sur le fluide qui s'écoule dans le seul canal autorisant son écoulement (le filtre hydrophobe 22 ne permettant pas au liquide de s'échapper), à savoir le canal 76 de réduction de débit. Au cours de son passage dans ce canal 76, le fluide voit son débit diminuer, par effet de perte de charge. Le fluide s'écoule ensuite dans le canal 32, puis dans la cavité 34 et dans le canal 36. Sous l'effet de la pression, le fluide soulève la membrane 16 qui passe alors en configuration de passage de liquide et peut ainsi s'écouler entre la membrane 16 et la surface d'appui 40, afin de passer dans le canal 60 et dans la cavité 61, et ainsi se présenter sous forme de goutte.

On notera que l'organe 20 de réduction de débit, étant une pièce à part, peut avoir une forme qui varie en fonction du liquide contenu dans le réservoir. Ainsi, le logement 30 est adapté pour recevoir différentes formes d'organe 20, telles que celles illustrée sur les figures 3a à 3f. Il est donc possible de fabriquer des lots comprenant des embouts présentant une même valve 16, un même support 14, un même capot 18, mais présentant des organes 20 différents.

L'embout 10 peut prendre des configurations différentes de celle illustrée sur la figure 1, telle que celle illustrée sur la figure 2.

L'embout de la figure 2 a des fonctionnalités analogues à celui de la figure 1, hormis le fait qu'il ne comporte pas de canal 42 de passage de l'air. Sur ce mode de réalisation, le réservoir 12 est de préférence un réservoir déformable de façon non élastique, si bien qu'il n'est pas nécessaire de faire passer de l'air pour combler la dépression générée par l'utilisateur lors de son appui sur le réservoir. Ainsi, dans cet exemple, le logement 30 est disposé au centre du support 14, et l'organe 20 de réduction de débit peut avoir un diamètre plus grand.

On notera que l'invention n'est pas limitée aux mode de réalisation précédemment décrits.

## Revendications

1. Dispositif de distribution de liquide, comprenant :
- un réservoir (12) pour le stockage du liquide à distribuer,
- un embout (10) de distribution monté sur le réservoir, muni d'un support (14) et d'une valve (16) de distribution de liquide, cette valve comprenant un matériau élastomère et pouvant prendre une configuration de blocage et une configuration de passage de liquide, par coopération avec le support (14),
- un organe (20) de réduction de débit, comprenant une forme (74) de déviation de liquide délimitant un canal (76) de réduction de débit, cet organe (20) de réduction de débit étant une pièce distincte du support (14) et distincte de la valve (16), **caractérisé par**
- le support (14) comprenant une partie d'étanchéité (38), portant une surface d'appui (40) de la valve (16) pour bloquer le passage de liquide en configuration de blocage, la partie d'étanchéité (38) ayant une forme sensiblement cylindrique et la surface d'appui (40) étant disposée à l'extrémité distale de cette forme cylindrique (38), et lavalve (16) comprenant une partie (58) sensiblement cylindrique, recouvrant la forme cylindrique (38) et délimitant avec cette forme cylindrique (38) un canal (36) de passage de liquide, agencé en aval du canal (76) de réduction de débit.

2. Dispositif selon la revendication précédente, dans lequel le support (14) comporte un logement (30) de réception de l'organe (20) de réduction de débit délimitant, avec ce denier, le canal (76) de réduction de débit.

3. Dispositif selon l'une quelconque des revendications précédentes, dans lequel l'organe (20) de réduction de débit a une forme sensiblement cylindrique, la forme de déviation (74) étant réalisée sur la périphérie extérieure de l'organe (20) de réduction de débit et comprenant une gorge hélicoïdale (74a, 74b).

4. Dispositif selon l'une quelconque des revendications 1 à 2, dans lequel l'organe (20) de réduction de débit a une forme sensiblement cylindrique, la forme de déviation étant réalisée sur la périphérie extérieure de l'organe de réduction de débit (20) et comprenant une gorge (74c, 74d) définissant des portions anguleuses.

5. Dispositif selon l'une quelconque des revendications 1 à 2, dans lequel l'organe (20) de réduction de débit a une forme sensiblement cylindrique, la forme de déviation étant réalisée sur la périphérie extérieure de l'organe de réduction de débit (20) et comprenant une gorge (74e) rectiligne, de préférence sensiblement parallèle à l'axe du cylindre de l'organe de réduction de débit (20).

6. Dispositif selon l'une quelconque des revendications 1 à 2, dans lequel l'organe (20) de réduction de débit a une forme sensiblement cylindrique, la forme de déviation étant réalisée sur la périphérie extérieure de l'organe de réduction de débit (20) et comprenant un réseau de plusieurs gorges (74f, 74g, 74h) comprenant au moins un noeud (74i) de confluence des gorges (74f, 74g, 74h).

7. Dispositif selon l'une quelconque des revendications précédentes, dans lequel le support (14) comporte en outre un canal (42) de passage d'air dans le réservoir (12), muni d'un filtre hydrophobe (22).

8. Lot de deux dispositifs selon l'une quelconque des revendications précédentes, chacun des deux dispositifs présentant une valve (16) et un support (14) de même forme et un organe (20) de réduction de débit de forme différente.

## Patentansprüche

1. Vorrichtung zur Flüssigkeitsverteilung, umfassend:
- einen Behälter (12) zur Speicherung der zu verteilenden Flüssigkeit,
- einen Verteilungsansatz (10), der auf dem Behälter montiert ist, verswehen mit einer Stütze (14) und einem Ventil (16) zur Flüssigkeitsverteilung, wobei dieses Ventil ein Elastomermaterial umfasst und durch Zusammenwirken mit der Stütze (14) eine Verschlussposition und eine Position für den Flüssigkeitsdurchfluss einnehmen kann,
- ein Element (20) zur Verringerung der Durchflussmenge, umfassend eine Form (74) zur Abzweigung der Flüssigkeit, die einen Kanal (76) zur Verringerung der Durchflussmenge begrenzt, wobei dieses Element (20) zur Verringerung der Durchflussmenge ein von der Stütze (14) und dem Ventil (16 getrenntes Teil ist,
**gekennzeichnet durch**
- die Stütze (14), umfassend einen Abdichtungsteil (38), der eine Auflagefläche (40) für das Ventil (16) trägt, um den Flüssigkeitsdurchfluss in der Verschlussposition zu blockieren, wobei der Abdichtungsteil (38) eine im Wesentlichen zylindrische Form hat und wobei die Stützfläche (40) am distalen Ende dieser zylindrischen Form (38) angeordnet ist, und wobei das Ventil (16) einen im Wesentlichen zylindrischen Teil (58) umfasst, der die zylindrische Form (38) abdeckt und mit dieser zylindrischen Form (38) einen Kanal (36) für den Flüssigkeitsdurchfluss begrenzt, der stromabwärts zu dem Kanal (76) zur Verringerung der Durchflussmenge angeordnet ist.

2. Vorrichtung nach dem vorhergehenden Anspruch, bei der die Stütze (14) eine Lagerung (30) für die Aufnahme des Elements (20) zur Verringerung der Durchflussmenge umfasst, die mit diesem letztgenannten einen Kanal (76) zur Verringerung der Durchflussmenge begrenzt.

3. Vorrichtung nach einem der vorhergehenden Ansprüche, bei der das Element (20) zur Verringerung der Durchflussmenge eine im Wesentlichen zylindrische Form hat, wobei die Abzweigungsform (74) an der äußeren Peripherie des Elements (20) zur Verringerung der Durchflussmenge ausgeführt ist und eine Spiralnut (74a, 74b) umfasst.

4. Vorrichtung nach einem der Ansprüche 1 bis 2, bei der das Element (20) zur Verringerung der Durchflussmenge eine im Wesentlichen zylindrische Form hat, wobei die Abzweigungsform an der äußeren Peripherie des Elements (20) zur Verringerung der Durchflussmenge ausgeführt ist und eine Nut (74c, 74d) umfasst, die Winkelabschnitte definiert.

5. Vorrichtung nach einem der Ansprüche 1 bis 2, bei dem das Element (20) zur Verringerung der Durchflussmenge eine im Wesentlichen zylindrische Form hat, wobei die Abzweigungsform an der äußeren Peripherie des Elements (20) zur Verringerung der Durchflussmenge ausgeführt ist und eine gerade Nut (74e) umfasst, die vorzugsweise im Wesentlichen parallel zur Achse des Zylinders des Elements (20) zur Verringerung der Durchflussmenge ist.

6. Vorrichtung nach einem der Ansprüche 1 bis 2, bei der das Element (20) zur Verringerung der Durchflussmenge eine im Wesentlichen zylindrische Form hat, wobei die Abzweigungsform an der äußeren Peripherie des Elements (20) zur Verringerung der Durchflussmenge ausgeführt ist und ein Netz von mehreren Nuten (74f, 74g, 74h) aufweist, umfassend mindestens einen Knoten (74i), an dem die Nuten (74f, 74g, 74h) zusammenkommen.

7. Vorrichtung nach einem der vorhergehenden Ansprüche, bei der die Stütze (14) ferner einen Kanal (42) für den Luftdurchfluss in den Behälter (12), der mit einem hydrophoben Filter (22) versehen ist, umfasst.

8. Satz von zwei Vorrichtungen nach einem der vorhergehenden Ansprüche, wobei jede der beiden Vorrichtungen ein Ventil (16) und eine Stütze (14) derselben Form und ein Element (20) zur Verringerung der Durchflussmenge von unterschiedlicher Form umfasst.

## Claims

1. Liquid dispenser device comprising:
· a container (12) for storing the liquid to be dispensed;
· a dispenser endpiece (10) mounted on the container, being provided with a support (14) and a liquid dispenser valve (16), the valve comprising an elastomer material and being capable of taking up a blocking configuration and a liquid-passing configuration by co-operating with the support (14); and
· a flowrate-reducer member (20) comprising a liquid-deflector shape (74) defining a flowrate-reducer channel (76), the flowrate-reducer member (20) being a part that is separate from the support (14) and separate from the valve (16), **characterised in that** the support (14) includes a sealing portion (38) carrying a bearing surface (40) of the valve (16) for blocking the passage of liquid in the blocking configuration, the sealing portion (38) being substantially cylindrical in shape and the bearing surface (40) being placed at the distal end of said cylindrical shape (38), and the valve (16) includes a substantially cylindrical portion (58) covering the cylindrical shape (38) and co-operating with the cylindrical shape (38) to define a liquid-passing channel (36) arranged downstream from the flowrate-reducer channel (76).

2. Device according to the preceding claim, wherein the support (14) includes a housing (30) for receiving the flowrate-reducer member (20) and co-operating therewith to define the flowrate-reducer channel (76).

3. Device according to any preceding claim, wherein the flowrate-reducer member (20) is substantially cylindrical in shape, the liquid-deflector shape (74) being made in the outer periphery of the flowrate-reducer member (20) and comprising a helical groove (74a, 74b).

4. Device according to claim 1 or claim 2, wherein the flowrate-reducer member (20) is substantially cylindrical in shape, the liquid-deflector shape being made in the outer periphery of the flowrate-reducer member (20) and comprising a groove (74c, 74d) defining angular portions.

5. Device according to claim 1 or claim 2, wherein the flowrate-reducer member (20) is substantially cylindrical in shape, the liquid-deflector shape being made in the outer periphery of the flowrate-reducer member (20) and comprising a rectilinear groove (74e), preferably substantially parallel to the cylinder axis of the flowrate-reducer member (20).

6. Device according to claim 1 or claim 2, wherein the flowrate-reducer member (20) is substantially cylindrical in shape, the liquid-deflector shape being made in the outer periphery of the flowrate-reducer member (20) and comprising a network of a plurality of grooves (74f, 74g, 74h) including at least one node (74i) where grooves (74f, 74g, 74h) meet.

7. Device according to any preceding claim, wherein the support (14) also includes an air-passing channel (42) for passing air into the container (12) and fitted with a hydrophobic filter (22).

8. Batch of two devices according to any preceding claim, the two devices presenting respective valves (16) and supports (14) of the same shape and flowrate-reducer members (20) of different shapes.
